# EUROPÄISCHE PATENTSCHRIFT

(11) **EP 1 123 336 B1**
(45) Veröffentlichungstag und Bekanntmachung des Hinweises auf die Patenterteilung: **10.03.2004**
(21) Anmeldenummer: 99944575.2
(22) Anmeldetag: 01.09.1999
(51) Int. Cl.: C08G 61/02, C07C 43/192, C07F 5/04

(54) **KONJUGIERTE POLYMERE, ENTHALTEND SPEZIELLE FLUOREN-BAUSTEINE MIT VERBESSERTEN EIGENSCHAFTEN**
CONJUGATED POLYMERS CONTAINING SPECIAL FLUORENE STRUCTURAL ELEMENTS WITH IMPROVED PROPERTIES
POLYMERES CONJUGUES CONTENANT DES ELEMENTS STRUCTURAUX FLUORENE SPECIAUX, A PROPRIETES AMELIOREES

(30) Priorität: 10.10.1998 DE 19846766
(43) Veröffentlichungstag der Anmeldung: 16.08.2001
(73) Patentinhaber: Covion Organic Semiconductors GmbH, 65926 Frankfurt (DE)
(72) Erfinder: SPREITZER, Hubert, D-65929 Frankfurt (DE); BECKER, Heinrich, D-61479 Glashütten (DE); KREUDER, Willi, D-55126 Mainz (DE)
(74) Vertreter: Luderschmidt, Schüler & Partner GbR
(86) Internationale Anmeldenummer: PCT/EP1999/006420
(87) Internationale Veröffentlichungsnummer: WO 2000/022026

(56) Entgegenhaltungen:
- WO-A-97/05184
- WO-A-97/33323
- WO-A-99/54943
- US-A- 5 777 070

## Beschreibung

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeige- und Beleuchtungselementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), in Gebrauch.

Neben anorganischen sind seit etwa 30 Jahren auch niedermolekulare organische Elektrolumineszenzmaterialien und -vorrichtungen bekannt (siehe z.B. US-A-3,172,862). Bis vor kurzem waren aber solche Vorrichtungen in ihrer praktischen Verwendbarkeit stark eingeschränkt.

In WO 90/13148 und EP-A-0,443,861 sind Elektrolumineszenzvorrichtungen beschrieben, die einen Film aus einem konjugierten Polymer als lichtemittierende Schicht (Halbleiterschicht) enthalten. Solche Vorrichtungen bieten zahlreiche Vorteile wie die Möglichkeit, großflächige, flexible Displays einfach und kostengünstig herzustellen. Im Gegensatz zu Flüssigkristalldisplays sind Elektrolumineszenzdisplays selbstleuchtend und benötigen daher keine zusätzliche rückwärtige Beleuchtungsquelle.

Eine typische Vorrichtung nach WO 90/13148 besteht aus einer lichtemittierenden Schicht in Form eines dünnen, dichten Polymerfilms (Halbleiterschicht), der wenigstens ein konjugiertes Polymer enthält. Eine erste Kontaktschicht steht in Kontakt mit einer ersten Oberfläche, eine zweite Kontaktschicht mit einer weiteren Oberfläche der Halbleiterschicht. Der Polymerfilm der Halbleiterschicht hat eine genügend geringe Konzentration von extrinsischen Ladungsträgern, so daß beim Anlegen eines elektrischen Feldes zwischen den beiden Kontaktschichten Ladungsträger in die Halbleiterschicht eingebracht werden, wobei die eine Kontaktschicht positiv gegenüber der anderen wird, und die Halbleiterschicht Strahlung aussendet. Die in solchen Vorrichtungen verwendeten Polymere sind konjugiert. Unter konjugiertem Polymer versteht man ein Polymer, das ein delokalisiertes Elektronensystem entlang der Hauptkette besitzt. Das delokalisierte Elektronensystem verleiht dem Polymer Halbleitereigenschaften und gibt ihm die Möglichkeit, positive und/oder negative Ladungsträger mit hoher Mobilität zu transportieren.

Für die Verwendung in EL-Elementen gemäß WO 90/13148 sind bereits sehr viele verschiedene Polymere vorgeschlagen worden. Besonders gut geeignet scheinen dabei Derivate des Poly(p-phenylen-vinylens) PPV zu sein. Derartige Polymere sind beispielsweise in WO 98/27136 beschrieben. Diese Polymere sind insbesondere für Elektrolumineszenz im grünen bis roten Spektralbereich geeignet. Im blauen bis blaugrünen Spektralbereich sind bisher hauptsächlich Polymere auf Basis des Polyp-phenylens (PPP) bzw. Polyfluorens (PF) vorgeschlagen worden. Entsprechende Polymere sind beispielsweise in EP-A-0,707,020, WO 97/05184 und WO 97/33323 beschrieben. Diese Polymere zeigen bereits gute EL-Eigenschaften, wobei die Entwicklung noch lange nicht abgeschlossen ist. So weisen Polymere im blauen bis blaugrünen Spektralbereich häufig noch das Phänomen der morphologischen Instabiltät auf. Z. B. zeigen viele Polyfluorene flüssigkristallines oder verwandtes Verhalten, welches im dünnen Film zu Domänenbildung führen kann was wiederum zur Herstellung einer homogen leuchtenden Fläche ungeeignet ist. Auch neigen diese Polymere zur Aggregatbildung, was die Elektrolumineszenz ungewünscht in den langwelligen Bereich verschiebt, sowie die Lebensdauer der EL-Elemente negativ beeinflußt.

Aufgabe der vorliegenden Erfindung war es daher, Polymere bereitzustellen, die im blauen und blaugrünen Spektralbereich zur Emission geeignet sind und die gleichzeitig verbessertes morphologisches Verhalten aufweisen.

Überraschend wurde nun gefunden, daß durch die Wahl spezieller Substitutionsmuster in ansonsten typischen Polymeren, die hauptsächlich durch 2,7-Fluorenylbausteine aufgebaut sind, die morphologischen Eigenschaften signifikant verbessert werden ohne die guten Anwendungseigenschaften (Emissionsfarbe, Quantenausbeute der Emission, Verwendbarkeit in EL-Applikationen) zu verlieren.

Die erfindungsgemäßen Polymere enthalten Fluoreneinheiten, die durch ihr Substitutionsmuster geeignet sind, Aggregation im Film zu unterdrücken. Dies wird im besonderen dadurch erreicht, daß die 9,9-Position durch zwei verschiedenartige Aromaten substituiert ist. Dieses Ergebnis ist überraschend, vorallem im Hinblick auf Angaben in der wissenschaftlichen Literatur (G. Klämer et al., Adv. Mater. 1998, 10, 993), wonach der Einbau von Diphenylfluoreneinheiten in der Hauptkette keine derartigen Effekte ergibt. Dies bedeutet aber gerade auch, daß es sich als besonders günstig erwiesen hat, zwei unterschiedliche aromatische Substituenten an dieser Position einzuführen.

Gegenstand der Erfindung sind konjugierte Polymere, die Struktureinheiten der Formel (I), 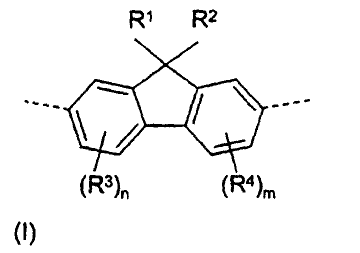 worin
- R¹, R²: zwei verschiedene Substituenten aus der Gruppe C₂-C₄₀-Hetereoaryl, C₅-C₄₀-Aryl darstellen; wobei die vorstehend genannten Aryle und/oder Heteroaryle mit einem oder mehreren Substituenten R³ substituiert sein können; verschiedenartig sollen die Aryle und/oder Heteroaryle im Sinne dieser Erfindung bereits sein, wenn sie sich durch die Art oder Stellung von Substituenten unterscheiden,
- R³, R⁴: gleich oder verschieden C₁-C₂₂-Alkyl, C₂-C₂₀-Hetereoaryl, C₅-C₂₀-Aryl, F, Cl, CN, SO₃R⁵, NR⁵R⁶; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein können, wobei die vorstehend genannten Aryle mit einem oder mehreren nichtaromatischen Substituenten R³ substituiert sein können,
- R⁵, R⁶: gleich oder verschieden H, C₁-C₂₂-Alkyl, C₂-C₂₀-Hetereoaryl, C₅-C₂₀-Aryl, dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein, wobei die vorstehend genannten Aryle mit einem oder mehreren nichtaromatischen Substituenten R³ substituiert sein können, und
- m, n: jeweils eine ganze Zahl 0, 1, 2 oder 3, bevorzugt 0 oder 1 ist, enthalten.

Bevorzugt stehen R¹, R² für zwei verschiedene Substituenten aus der Gruppe C₅-C₄₀-Aryl, C₂-C₄₀-Heteroaryl; wobei die vorstehend genannten Aryle bzw. Heteroaryle mit einem oder mehreren Substituenten R³ substituiert sein können.

Das erfindungsgemäße Polymer enthält mindestens 10 Mol-%, vorzugsweise 10 Mol-% bis 100 Mol-%, an Struktureinheiten der Formel (I) statistisch, alternierend, periodisch, oder in Blöcken eingebaut.

Die erfindungsgemäßen Polymere sind vorzugsweise Copolymere bestehend aus einer oder mehrerer Struktureinheiten der Formel (I). In einer weiteren Ausführungsform der vorliegenden Erfindung kann das erfindungsgemäße Polymer auch verschiedene Struktureinheiten der Formel (I) und weitere - für sich alleine nicht erfindungsgemäße - Struktureinheiten enthalten. Beispiele für derartige weitere Monomere sind 1,4-Phenylene, 4,4'-Biphenyle und weitere 2,7-Fluorene, die gegebenenfalls auch Substituenten tragen können, bevorzugt dabei verzweigte oder unverzweigte C₁-C₂₂-Alkyl- oder Alkoxygruppen.
Die erfindungsgemäßen Polymere weisen im allgemeinen 10 bis 10000, vorzugsweise 10 bis 5000, besonders bevorzugt 50 bis 5000, ganz besonders bevorzugt 50 bis 1000 Wiederholeinheiten auf.
Besonders bevorzugt sind Polymere bei denen m, n gleich null sind.

Die erfindungsgemäßen Polymere lassen sich durch die unterschiedlichsten Reaktionen aufbauen. Bevorzugt sind jedoch einheitliche C-C-Kupplungsreaktion, z. B. Suzuki-Kondensation und Stille-Kondensation. Einheitliche C-C-Kupplungsreaktion soll in diesem Zusammenhang bedeuten, daß aus der Stellung der reaktiven Gruppen in den entsprechenden Monomeren die Verknüpfung in den Polymeren festgelegt ist. Dies ist besonders gut durch die o. g. Reaktionen, die sich wegen des sauberen Ablaufs sehr gut eignen, gegeben. Geeignet ist weiterhin die Nickel katalysierte Kupplung von Halogenaromaten (Yamamoto-Kupplung). Weniger gut sind hingegen oxidative Verfahren (z. B. Oxidative Kupplung mit Fe(III)-Salzen) geeignet, da diese zu undefinierten Verknüpfungen führen.
Aus oben gesagtem resultiert auch die bevorzugte Wahl der Monomere: diese stellen die entsprechenden Bishalogen-, Bispseudohalogen- (d. h. im Sinne dieser Erfindung z. B. Bis-Triflat, Bis-Nonaflat, Bis-Tosylat), Bisboronsäure-, Bisstannat-, Monohalogen-monoboronsäure-, Monohalogen-monostannatderivate der Verbindungen gemäß Formel (I) und Formel (II) dar.

Die Synthese der erfindungsgemäßen Polymere ist beispielhaft durch das nachfolgende Schema 1 wiedergegeben:

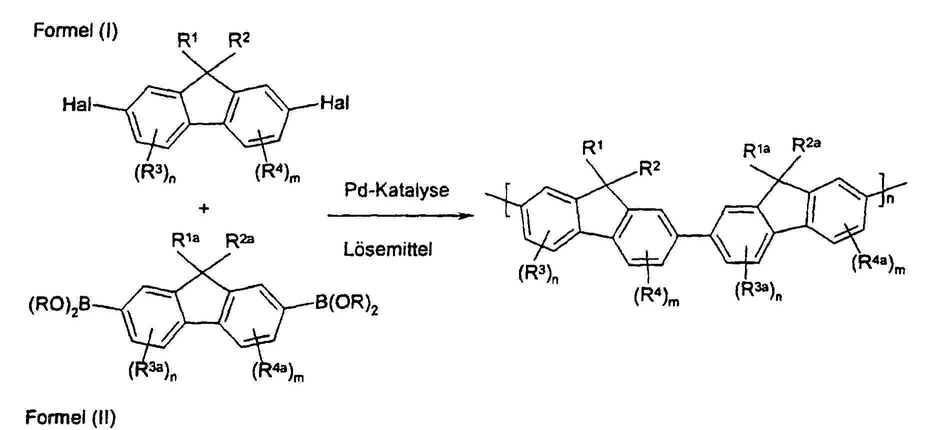

Der Rest R bedeutet im vorstehenden Schema Wasserstoff oder einen beliebigen organischen Rest, vorzugsweise einen Rest mit 1 bis 40 Kohlenstoffatomen. Beispiele hierfür sind entsprechende Alkylreste, z. B. Methyl oder Butyl. Des weiteren kann R für einen aromatischen Rest mit 5 bis 30 Kohlenstoffatomen stehen, der gegebenenfalls substituiert sein kann. Der Rest R^{1a} entspricht in seiner Definition dem Rest R¹; Der Rest R^{2a} entspricht in seiner Definition dem Rest R²; Der Rest R^{3a} entspricht in seiner Definition dem Rest R³; Der Rest R^{4a} entspricht in seiner Definition dem Rest R⁴.
In Schema 1 ist die Polymerisation via Suzuki-Kupplung angegeben. Es sei ausdrücklich darauf verwiesen, daß es sich hierbei nur um eine mögliche Ausführungsform handelt. Es sind natürlich auch andere Kombinationen von Boronsäuren und Halogenen/Pseudohalogenen ausführbar. Analog ist mit entsprechenden Zinnverbindungen auch die Polymerisation nach Stille durchzuführen.

Die Polymerisation gemäß Suzuki ist wie folgt vorzunehmen:
Die der Struktureinheit der Formel (I) zugrundeliegenden Monomere (und gegebenenfalls weitere zusätzliche Monomere mit entsprechenden aktiven Abgangsgruppen) werden in einem inerten Lösungsmittel bei einer Temperatur im Bereich von 0°C bis 200°C in Gegenwart eines Palladiumkatalysators zur Reaktion gebracht. Dabei ist darauf zu achten, daß die Gesamtheit aller verwendeten Monomere ein möglichst ausgeglichenes Verhältnis an Boronsäurefunktionen zu Halogen- bzw. Pseudohalogenfunktionen aufweist. Es kann sich zudem als vorteilhaft erweisen, am Ende der Reaktion durch Endcapping mit monofunktionellen Reagenzien eventuell überschüssige reaktive Gruppen zu entfernen.

Zur Durchführung der angegebenen Reaktion mit Boronsäure(ester)n werden die aromatischen Borverbindungen, die aromatischen Halogenverbindungen, eine Base und katalytische Mengen des Palladiumkatalysators in Wasser oder in ein oder mehrere inerte organische Lösungsmittel oder vorzugsweise in eine Mischung aus Wasser und einem oder mehreren inerten organischen Lösungsmitteln gegeben und bei einer Temperatur von 0 bis 200°C, bevorzugt bei 30 bis 170°C, besonders bevorzugt bei 50 bis 150°C, insbesonders bevorzugt bei 60 bis 120°C für einen Zeitraum von 1 h bis 200 h, bevorzugt 5 h bis 150 h, besonders bevorzugt 24 h bis 120 h, gerührt. Es kann sich dabei auch als vorteilhaft erweisen, eine Art von Monomer (z. B. ein Bisboronsäurederivat) kontinuierlich oder diskontinuierlich über einen längeren Zeitraum langsam zuzudosieren, um damit das Molekulargewicht zu regeln. Das Rohprodukt kann nach dem Fachmann bekannten und dem jeweiligen Polymer angemessenen Methoden, z.B. mehrfaches Umfällen bzw. auch durch Dialyse gereinigt werden.

Für das beschriebene Verfahren geeignete organische Lösungsmittel sind beispielsweise Ether, z. B. Diethylether, Dimethoxyethan, Diethylenglykoldimethylether, Tetrahydrofuran, Dioxan, Dioxolan, Diisopropylether, tert.-Butylmethylether, Kohlenwasserstoffe, z. B. Hexan, iso-Hexan, Heptan, Cyclohexan, Toluol, Xylol, Alkohole, z. B. Methanol, Ethanol, 1-Propanol, 2-Propanol, Ethylenglykol, 1-Butanol, 2-Butanol, tert.-Butanol, Ketone, z. B. Aceton, Ethylmethylketon, iso-Butylmethylketon, Amide, z.B. Dimethylformamid, Dimethylacetamid, N-Methylpyrrolidon, Nitrile, z.B. Acetonitril, Propionitril, Butyronitril, und Mischungen derselben.

Bevorzugte organische Lösungsmittel sind Ether, wie Dimethoxyethan, Diethylenglykoldimethylether, Tetrahydrofuran, Dioxan, Diisopropylether, t-Butylmethylether, Kohlenwasserstoffe, wie Hexan, Heptan, Cyclohexan, Toluol, Xylol, Alkohole, wie Methanol, Ethanol, 1-Propanol, 2-Propanol, 1-Butanol, 2-Butanol, tert.-Butanol, Ethylenglykol, Ketone, wie Ethylmethylketon, iso-Butylmethylketon, Amide, wie Dimethylformamid, Dimethylacetamid, N-Methylpyrrolidon und Mischungen derselben.

Besonders bevorzugte Lösungsmittel sind Ether, z. B. Dimethoxyethan, Tetrahydrofuran, Kohlenwasserstoffe, z. B. Cyclohexan, Toluol, Xylol, Alkohole, z. B. Ethanol, 1-Propanol, 2-Propanol, 1-Butanol, tert.-Butanol und Mischungen derselben.

In einer besonders bevorzugten Variante werden bei dem beschriebenen Verfahren Wasser und ein oder mehrere Lösungsmittel eingesetzt. Beispiele sind Mischungen aus Wasser und Toluol, Wasser, Toluol und Tetrahydrofuran sowie Wasser, Toluol und Ethanol.

Basen, die bei dem beschriebenen Verfahren vorzugsweise Verwendung finden sind Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine.

Besonders bevorzugt sind Alkali- und Erdalkalimetallhydroxide, Alkali- und Erdalkalimetallcarbonate und Alkalimetallhydrogencarbonate.
Insbesondere bevorzugt sind Alkalimetallhydroxide, wie Natriumhydroxid und Kaliumhydroxid, sowie Alkalimetallcarbonate und Alkalimetallhydrogencarbonate, wie Lithiumcarbonat, Natriumcarbonat und Kaliumcarbonat.

Die Base wird bei dem angegebenen Verfahren bevorzugt mit einem Anteil von 100 bis 1000 Mol-%, besonders bevorzugt 100 bis 500 Mol-%, ganz besonders bevorzugt 150 bis 400 Mol-%, insbesondere 180 bis 250 Mol-%, bezogen auf Borgruppen, eingesetzt.

Der Palladiumkatalysator enthält Palladiummetall oder eine Palladium (0) oder (II) Verbindung und einen Komplexliganden, vorzugsweise einen Phosphanliganden. Die beiden Komponenten können eine Verbindung bilden, z.B. das besonders bevorzugte Pd(PPh₃)₄, oder getrennt eingesetzt werden.

Als Palladiumkomponente eignen sich beispielsweise Palladiumverbindungen, wie Palladiumketonate, Palladiumacetylacetonate, Nitrilpalladiumhalogenide, Olefinpalladiumhalogenide, Palladiumhalogenide, Allylpalladiumhalogenide und Palladiumbiscarboxylate, bevorzugt Palladiumketonate, Palladiumacetylacetonate, bis-η²-Olefinpalladiumdihalogenide, Palladium(II)halogenide, η³-Allylpalladiumhalogenid Dimere und Palladiumbiscarboxylate, ganz besonders bevorzugt Bis(dibenzylidenaceton)palladium(0) [Pd(dba)₂)], Pd(dba)₂ CHCl₃, Palladiumbisacetylacetonat, Bis(benzonitril)palladiumdichlorid, PdCl₂, Na₂PdCl₄, Dichlorobis(dimethylsulfoxid)palladium(II), Bis(acetonitril)palladiumdichlorid, Palladium-II-acetat, Palladium-II-propionat, Palladium-II-butanoat und (1c,5c-Cyclooctadien)palladiumdichlorid.

Ebenso als Katalysator dienen kann Palladium in metallischer Form, im folgenden nur Palladium genannt, vorzugsweise Palladium in pulverisierter Form oder auf einem Trägermaterial, z.B. Palladium auf Aktivkohle, Palladium auf Aluminiumoxid, Palladium auf Bariumcarbonat, Palladium auf Bariumsulfat, Palladium auf Aluminiumsilikaten, wie Montmorillonit, Palladium auf SiO₂ und Palladium auf Calciumcarbonat, jeweils mit einem Palladiumgehalt von 0,5 bis 10 Gew.-%. Besonders bevorzugt sind Palladium in kolloidaler oder pulverisierter Form, Palladium auf Aktivkohle, Palladium auf Barium- und/oder Calciumcarbonat und Palladium auf Bariumsulfat, jeweils mit einem Palladiumgehalt von 0,5 bis 10 Gew.-% insbesondere bevorzugt ist Palladium auf Aktivkohle mit einem Palladiumgehalt von 5 oder 10 Gew.-%
Der Palladiumkatalysator wird bei dem erfindungsgemäßen Verfahren mit einem Anteil von 0,01 bis 10 Mol-%, bevorzugt 0,05 bis 5 Mol-%, besonders bevorzugt 0,1 bis 3 Mol-%, insbesondere bevorzugt 0,1 bis 1,5 Mol-%, bezogen auf die Halogengruppen, eingesetzt.

Für das Verfahren geeignete Liganden sind beispielsweise Phosphane, wie Trialkylphosphane, Tricycloalkylphosphane, Triarylphosphane, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können.
Beispiele für im Rahmen des hier beschriebenen Verfahrens verwendbare Phosphane sind Trimethylphosphan, Tributylphosphan, Tricyclohexylphosphan, Triphenylphosphan, Tritolylphosphan, Tris-(4-dimethylaminophenyl)phosphan, Bis(diphenylphosphano)methan, 1,2-Bis(diphenylphosphano)ethan, 1,3-Bis(diphenylphosphano)propan und 1,1'-Bis(diphenylphosphano)ferrocen.
Weitere geeignete Liganden sind beispielsweise Diketone, z. B. Acetylaceton und Octafluoracetylaceton und tert. Amine, z. B. Trimethylamin, Triethylamin, Tri-n-propylamin und Triisopropylamin.
Bevorzugte Liganden sind Phosphane und Diketone, besonders bevorzugt sind Phosphane.
Ganz besonders bevorzugte Liganden sind Triphenylphosphan, 1,2-Bis(diphenylphosphano)ethan, 1,3-Bis(diphenylphosphano)propan und 1,1'-Bis(diphenylphosphano)ferrocen, insbesondere Triphenylphosphan.
Für das Verfahren weiterhin geeignet sind wasserlösliche Liganden, die beispielsweise Sulfonsäuresalz- und/oder Sulfonsäurereste und/oder Carbonsäuresalz- und/oder Carbonsäurereste und/oder Phosphonsäuresalz und/oder Phosphonsäurereste und/oder Phosphoniumgruppen und/oder Peralkylammoniumgruppen und/oder Hydroxygruppen und/oder Polyethergruppen mit geeigneter Kettenlänge enthalten.

Bevorzugte Klassen von wasserlöslichen Liganden sind mit den obigen Gruppen substituierte Phosphane, wie Trialkylphosphane, Tricycloalkylphosphane, Triarylphosphane, Dialkylarylphosphane, Alkyldiarylphosphane und Heteroarylphosphane wie Tripyridylphosphan und Trifurylphosphan, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können, Phosphite, Phosphinigsäureester und Phosphonigsäureester, Phosphole, Dibenzophosphole und Phosphoratome enthaltende cyclische bzw. oligo- und polycyclische Verbindungen.

Der Ligand wird bei dem Verfahren mit einem Anteil von 0,1 bis 20 Mol %, bevorzugt 0,2 bis 15 Mol %, besonders bevorzugt 0,5 bis 10 Mol %, insbesonders bevorzugt 1 bis 6 Mol %, bezogen auf die aromatischen Halogengruppen, eingesetzt. Es können gegebenenfalls auch Mischungen zweier oder mehrerer verschiedener Liganden eingesetzt werden.

Vorteilhafte Ausführungsformen des beschriebenen Verfahrens der Suzuki-Variante sind für niedermolekulare Kupplungen z.B. in WO 94/101 05, EP-A-679 619, EP-A-694 530 und EP-A-839 173 beschrieben, auf die hiermit ausdrücklich Bezug genommen wird. Sie gelten durch Zitat als Bestandteil der Beschreibung dieser Anmeldung.

Die Polymerisation gemäß Stille ist wie folgt vorzunehmen:
Die den Struktureinheiten der Formel (I) und (II) zugrundeliegenden Monomeren (und gegebenenfalls weitere Monomere mit entsprechenden aktiven Abgangsgruppen) werden in einem inerten Lösungsmittel bei einer Temperatur im Bereich von 0°C bis 200°C in Gegenwart eines Palladiumkatalysators zur Reaktion gebracht. Dabei ist darauf zu achten, daß die Gesamtheit aller verwendeten Monomere ein möglichst ausgeglichenes Verhältnis an Zinnorganylfunktionen zu Halogen- bzw. Pseudohalogenfunktionen aufweist. Es kann sich zudem als vorteilhaft erweisen, am Ende der Reaktion durch Endcapping mit monofunktionellen Reagenzien eventuell überschüssige reaktive Gruppen zu entfernen.

Ein Überblick über diese Reaktion findet sich z.B. bei J.K. Stille, Angew. Chemie Int. Ed. Engt. 1986, 25, 508.

Zur Durchführung des Verfahrens werden bevorzugt aromatische Zinnverbindungen, aromatische Halogenverbindungen, in ein oder mehrere inerte organische Lösungsmittel gegeben und bei einer Temperatur von 0°C bis 200°C, bevorzugt bei 30°C bis 170°C, besonders bevorzugt bei 50°C bis 150°C, insbesondere bevorzugt bei 60°C bis 120°C für einen Zeitraum von 1 h bis 200 h, bevorzugt 5 h bis 150 h, besonders bevorzugt 24 h bis 120 h, gerührt. Es kann sich dabei auch als vorteilhaft erweisen, eine Art von Monomer (z. B. ein Bisstannylderivat) kontinuierlich oder diskontinuierlich über einen längeren Zeitraum langsam zuzudosieren, um damit das Molekulargewicht zu regeln. Das Rohprodukt kann nach dem Fachmann bekannten und dem jeweiligen Polymer angemessenen Methoden, z.B. mehrfaches Umfällen bzw. auch durch Dialyse gereinigt werden.

Für das beschriebene Verfahren geeignete organische Lösungsmittel sind beispielsweise Ether, z.B. Diethylether, Dimethoxyethan, Diethylenglykoldimethylether, Tetrahydrofuran, Dioxan, Dioxolan, Diisopropylether, tert.-Butylmethylether, Kohlenwasserstoff, z.B. Hexan, iso-Hexan, Heptan, Cyclohexan, Benzol, Toluol, Xylol, Alkohole, z.B. Methanol, Ethanol, 1-Propanol, 2-Propanol, Ethylenglykol, 1-Butanol, 2-Butanol, tert.-Butanol, Ketone, z.B. Aceton, Ethylmethylketon, iso-Butylmethylketon, Amide, z.B. Dimethylformamid (DMF), Dimethylacetamid, N-Methylpyrrolidon, Nitrile, z.B. Acetonitril, Propionitril, Butyronitril und Mischungen derselben.

Bevorzugte organische Lösungsmittel sind Ether, wie Dimethoxyethan, Diethylenglykoldimethylether, Tetrahydrofuran, Dioxan, Diisopropylether, Kohlenwasserstoffe, wie Hexan, Heptan, Cyclohexan, Benzol, Toluol, Xylol, Alkohole, wie Methanol, Ethanol, 1-Propanol, 2-Propanol, 1-Butanol, 2-Butanol, tert.-Butanol, Ethylenglykol, Ketone, wie Ethylmethylketon, oder Amide, wie DMF.

Besonders bevorzugte Lösungsmittel sind Amide, ganz besonders bevorzugt ist DMF.

Die Palladium- und die Phosphinkomponente sind analog zu der Beschreibung für die Suzuki-Variante zu wählen.

Weiterer Gegenstand der vorliegenden Erfindung sind die dem erfindungsgemäßen Polymeren zugrundliegenden monomeren Vorprodukte. Diese werden durch die Formel (A) 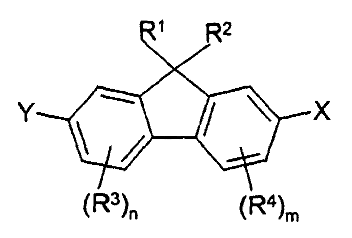 worin
- R¹, R²: zwei verschiedene Substituenten aus der Gruppe C₂-C₄₀-Hetereoaryl, C₅-C₄₀-Aryl darstellen; wobei die vorstehend genannten Aryle bzw. Heteroaryle mit einem oder mehreren Substituenten R³ substituiert sein können; verschiedenartig sollen die Aryle bzw. Heteroaryle im Sinne dieser Erfindung bereits sein, wenn sie sich durch die Art oder Stellung von Substituenten unterscheiden:
- R³, R⁴: gleich oder verschieden C₁-C₂₂-Alkyl, C₂-C₂₀-Hetereoaryl, C₅-C₂₀-Aryl, F, Cl, CN, SO₃R⁵, NR⁵R⁶; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein können, wobei die vorstehend genannten Aryle mit einem oder mehreren nichtaromatischen Substituenten R³ substituiert sein können,
- R⁵, R⁶: gleich oder verschieden H, C₁-C₂₂-Alkyl, C₂-C₂₀-Hetereoaryl, C₅-C₂₀-Aryl; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein, wobei die vorstehend genannten Aryle mit einem oder mehreren nichtaromatischen Substituenten R³ substituiert sein können, und
- m, n: jeweils eine ganze Zahl 0, 1, 2 oder 3, bevorzugt 0 oder 1 ist,
- X, Y: gleich oder verschieden Halogen, bevorzugt Cl, Br oder I, B(OR⁷)₂ oder SnR⁷R⁸R⁹,
- R⁷, R⁸, R⁹: gleich oder verschieden H, C₁-C₆-Alkyl, wobei zwei Reste auch einen gemeinsamen Ring bilden können und diese Reste auch verzweigt oder unverzweigt sein können.

Beispielhafte Monomere sind im nachfolgenden Schema 2 aufgeführt:

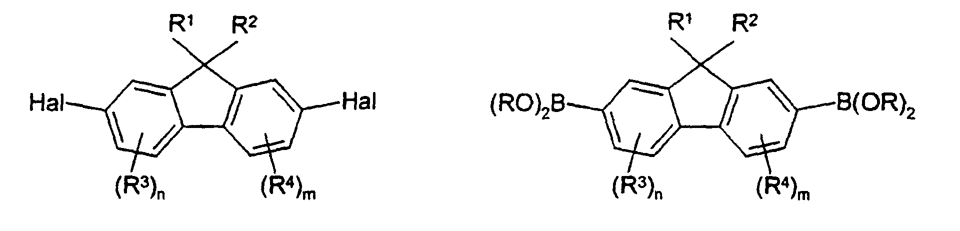

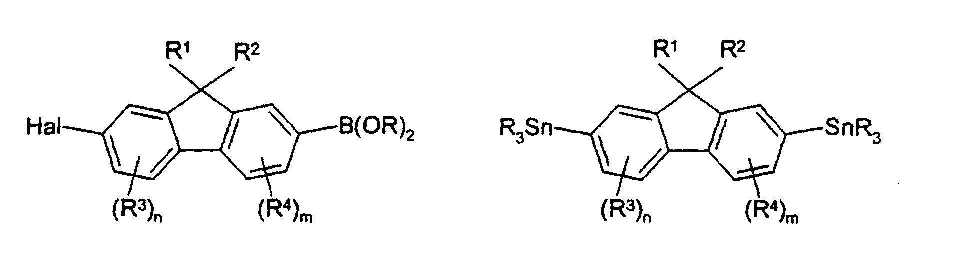

Die geeigneten 9-Aryl¹-9-aryl²-fluorenmonomere sind beispielhaft wie im folgenden Schema 3 beschrieben zu synthetisieren:

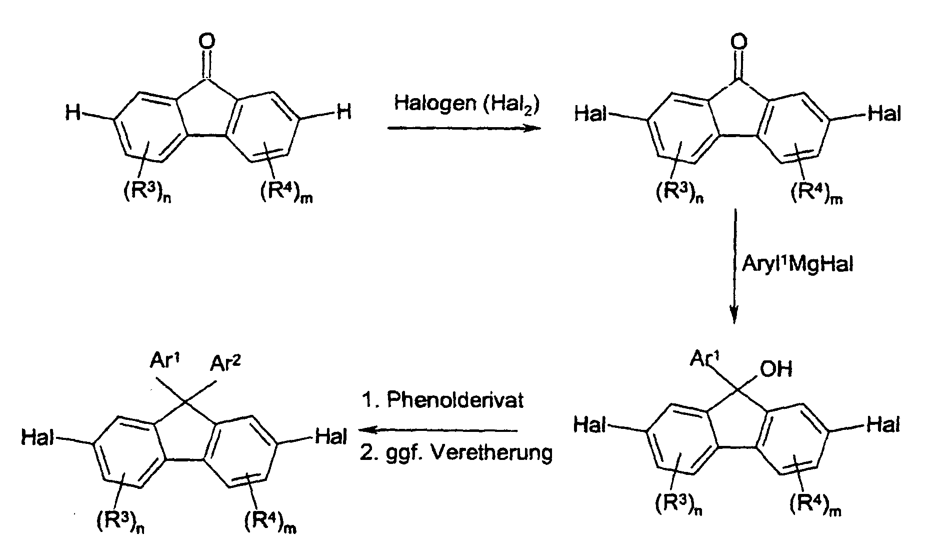

In den vorstehenden Formel steht Ar¹ für einen Rest R¹ und Ar² für einen Rest R².

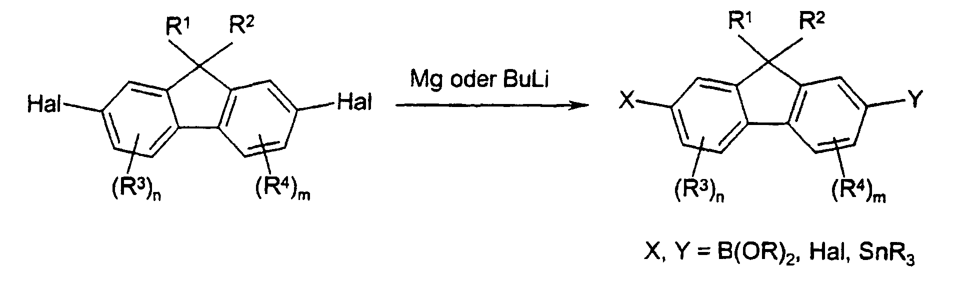

Der Rest R hat die gleiche Bedeutung wie vorstehend definiert (Schema 1 und 2) Demzufolge sind einfache Fluorenonderivate zu halogenieren. Für m, n = 0 entspricht dies der Halogenierung von Fluorenon. 2,7-Dibromfluorenon beispielsweise ist kommerziell erhältlich (z. B. Aldrich). Anschließend kann eine erste Arylgruppe durch die übliche Grignard-Reaktion eingeführt werden. Dies kann beispielsweise gemäß den Beschreibungen im Organikum (15. Auflage, 1977, Seite 623) geschehen.
Anschließend kann ein Phenolderivat sauer katalysiert addiert werden. Dies kann analog den Beschreibungen in WO 92/07812 geschehen. Die dadurch erhaltene Verbindung kann verethert werden. Dies kann z. B. der Williamson'schen Methode folgend geschehen (vgi. Organikum, 15. Auflage, 1977, Seite 253).

Die dadurch erhaltenen Verbindungen (Bishalogenfluorenderivate) sind bereits als Monomere verwendbar. Durch eine weitere Umsetzung (Metallierung mit anschließender Reaktion entweder mit Borsäureester oder Trialkylzinnhalogenid) sind weitere Monomere zu gewinnen: Fluorenbisboronsäurederivate, Fluorenbisstannate bzw. bei entsprechender Stöchiometrie auch Monohalogenfluorenmonoboronsäurederivate bzw. Monohalogenfluorenmonostannate. Diese letztgenannten Umsetzung können nach Standardverfahren durchgeführt werden, wie sie beispielsweise in WO 98/27136 beschrieben sind.

Eine andere Methode ist dem folgenden Schema zu entnehmen:

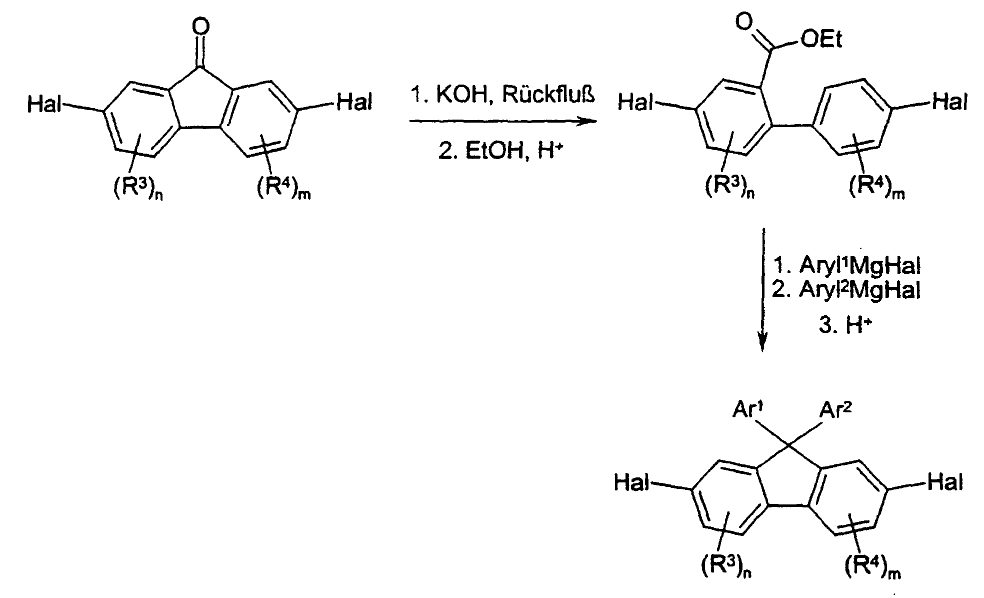

Für die Rest gilt hierbei Aryl¹ = Ar¹ = R¹ und Aryl² = Ar² = R².
Ausgehend von bishalogenierten Fluorenonderivaten (vgl. obige) können zunächst als Zwischenprodukt 4,4'-Dihalogenbiphenyl-2-carbonsäureesterderivate durch basische Ringöffnung mit anschließender Veresterung erhalten werden. Diese Verbindungen können dann durch Umsetzung mit zwei verschiedenen Aryl-Grignard-Reagenzien, wobei sich die zwischenzeitliche Hydrolyse, um das entsprechende Keton als intermediat zu isolieren, als hilfreich erwiesen hat, und anschließende saure Cyclisierung zu den gewünschten Fluorenmonomeren umgesetzt werden.
Wie schon oben beschrieben ist hier eine weitere Umsetzung zu den entsprechenden Fluorenbisboronsäurederivaten, Fluorenbisstannaten bzw. Monohalogenfluorenmonoboronsäurederivaten bzw. Monohalogenfluorenmonostannaten möglich.

Damit ist nun gezeigt, daß Monomere, die bevorzugt durch die oben beschriebenen Polymerisationsmethoden zu erfindungsgemäßen Polymeren umgesetzt werden können, leicht zugänglich sind.

Die so erhaltenen Polymere eigenen sich ganz besonders bevorzugt als organischer Halbleiter und insbesondere als Elektrolumineszenzmaterialien.

Als Elektrolumineszenzmaterialien im Sinne der Erfindung gelten Materialien, die als aktive Schicht in einer Elektrolumineszenzvorrichtung Verwendung finden können. Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht).

Gegenstand der Erfindung ist daher auch die Verwendung eines erfindungsgemäßen Polymers als Elektrolumineszenzmaterial sowie als organischer Halbleiter.

Um als Elektrolumineszenzmaterialien Verwendung zu finden, werden die erfindungsgemäßen Polymere im allgemeinen nach bekannten, dem Fachmann geläufigen Methoden, wie Eintauchen (Dipping) oder Lackschleudern (Spincoating), in Form eines Films auf ein Substrat aufgebracht.

Gegenstand der Erfindung ist somit ebenfalls eine Elektrolumineszenzvorrichtung mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht sein.
Der allgemeine Aufbau solcher Elektrolumineszenzvorrichtungen ist beispielsweise in US-A-4,539,507 und US-A-5,151,629 beschrieben. Polymere enthaltende Elektrolumineszenzvorrichtungen sind beispielsweise in WO 90/13148 oder EP-A-0,443,861 beschrieben.

Sie enthalten üblicherweise eine elektrolumineszierende Schicht zwischen einer Kathode und einer Anode, wobei mindestens eine der Elektroden transparent ist. Zusätzlich können zwischen der elektrolumineszierenden Schicht und der Kathode eine oder mehrere Elektroneninjektions- und/oder Elektronentransportschichten eingebracht sein und/oder zwischen der elektrolumineszierenden Schicht und der Anode eine oder mehrere Lochinjektions- und/oder Lochtransportschichten eingebracht sein. Als Kathode können vorzugsweise Metalle oder metallische Legierungen, z.B. Ca, Sr, Ba, Mg, Al, In, Mg/Ag dienen. Als Anode können Metalle, z.B. Au, oder andere metallisch leitende Stoffe, wie Oxide, z.B. ITO (Indiumoxid/Zinnoxid) auf einem transparentem Substrat, z.B. aus Glas oder einem transparenten Polymer, dienen.
Im Betrieb wird die Kathode auf negatives Potential gegenüber der Anode gesetzt. Dabei werden Elektronen von der Kathode in die Elektroneninjektionsschicht/Elektronentransportschicht bzw. direkt in die lichtemittierende Schicht injiziert. Gleichzeitig werden Löcher von der Anode in die Lochinjektionsschicht/Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht bzw. innerhalb der lichtemittierenden Schicht zu Elektronen/Loch-Paaren, die unter Aussendung von Licht rekombinieren. Die Farbe des emittierten Lichtes kann durch die als lichtemittierende Schicht verwendeten Materialien variiert werden.

Elektrolumineszenzvorrichtungen finden Anwendung z.B. als selbstleuchtende Anzeigeelemente, wie Kontrollampen, alphanumerische Displays, monochromen oder multichromen Matrixdisplays, Hinweisschilder, elektrooptischen Speichern und in optoelektronischen Kopplern.

In der vorliegenden Anmeldung sind verschiedene Dokumente zitiert, beispielsweise um das technische Umfeld der Erfindung zu illustrieren. Auf alle diese Dokumente wird hiermit ausdrücklich Bezug genommen, sie gelten durch Zitat als Bestandteil der vorliegenden Anmeldung.

Die Erfindung wird durch die Beispiele näher erläutert, ohne sie dadurch einschränken zu wollen.

### A) Synthese der Monomeren:

### 1. Herstellung der erfindungsgemäßen Monomeren:

### Beispiel M1: Darstellung von 2,7-Dibrom-9-(2,5-dimethylphenyl)-9-[4-(3,7-dimethyloctyloxy)phenyl]fluoren

### i) 2,7-Dibrom-9-(2,5-dimethylphenyl)fluoren-9-ol:

Das Grignardreagenz von 102 g Brom-p-xylol, dargestellt in üblicher Weise in THF, wurde zu einer Suspension von 2,7-Dibromfluorenon (169 g) in 500 ml THF bei 5-15°C zugetropft. Anschließend wurde der Ansatz für zwei Stunden zum Rückfluß erhitzt. Zur Hydrolyse wurde mit ca. 1200 ml Eiswasser und 30 ml konz. Schwefelsäure versetzt. Die Phasen wurden getrennt, die wäßrige Phase mehrfach mit Ethylacetat rückgeschüttelt und die vereinigten organischen Phasen nochmals mit Wasser rückgeschüttelt. Nach Trocknung über Na₂SO₄ wurde das Lösemittel abgezogen und das erhaltene Rohprodukt aus Ethanol umkristallisiert.
Ausbeute: 163 g (73%)
¹H NMR (d₆-DMSO): [ppm] δ= 8.05 (s (br), 1 H, OH), 7.85 (d, 2 H, H-4, J = 8 Hz), 7.60 (dd, 2 H, H-3, J₁ = 1 Hz, J₂ = 8 Hz), 7.15 (d, 2 H, H-1, J = 2 Hz), 7.02 (dd (br), 1 H, H-4', J₁ = 2 Hz, J₂ = 8 Hz), 6.85 (d, 1 H, H-3', J = 7.5 Hz), 6.48 (s (br), 1 H, H-6'), 2.38 (s, 3 H, Me), 1.2 (s(br), 3 H, Me).

### ii) 2,7-Dibrom-9-(2,5-dimethylphenyl)-9-(4-hydroxyphenyl)fluoren

9.5 g Phenol wurden mit 22.2 g 2,7-Dibrom-9-(2,5-dimethylphenyl)fluoren-9-ol, 25 ml Toluol und 0.1 ml Mercaptopropionsäure vermischt. Anschließend wurden 5 ml konz. Schwefelsäure zugetropft. Der Ansatz wurde dann für ca. 2 h bei 60°C gerührt, schließlich mit 100 ml MeOH und 100 ml Wasser versetzt. Der Feststoff wurde abgesaugt und durch Ausrühren mit Ethanol weiter gereinigt.
Ausbeute: 16 g (61 %)
¹H NMR (d₆-DMSO): [ppm] δ= 9.4 (s (br), 1 H, OH), 7.92 (d, 2 H, H-4, J = 8 Hz), 7.60 (dd, 2 H, H-3, J₁ = 1 Hz, J₂ = 8 Hz), 7.45 (d, 2 H, H-1, J = 2 Hz), 6.98 (m, 4 H, H-2", H-3', H-4'), 6.87 (s (br), 1 H, H-6'), 6.67 (Teil eines AA'BB', 2 H, H-3"), 2.17 (s, 3 H, Me), 1.38 (s(br), 3 H, Me).

### iii) 2,7-Dibrom-9-(2,5-dimethylphenyl)-9-[4-(3,7-dimethyloctyloxy)phenyl]fluoren

52 g 2,7-Dibrom-9-(2,5-dimethylphenyl)-9-(4-hydroxyphenyl)fluoren wurden mit 18 g 3,7-Dimethyloctylchlorid, 80 ml Ethanol, 7 g KOH, 1 g Nal für 5 Tage refluxiert. Nach DC war die Reaktion nach dieser Zeit beendet. Das Lösemittel wurde abgezogen, mit Ethylacetat versetzt und der Niederschlag wurde abgesaugt. Dieser wurde noch mehrfach mit Ethylacetat nachgewaschen. Die organische Phase wurde getrocknet und das Lösemittel wurde abgezogen.
Das Produkt wurde durch doppelte Destillation am Kurzwegverdampfer (10⁻³ mbar; 1. Destillation zur Trocknung: 80°C; 2. Destillation: 250°C) gereinigt.
Ausbeute: 48 g (73%)
¹H NMR (CDCl₃): [ppm] δ= 7.83 (d, 2 H, H-4, J = 8 Hz), 7.55 (dd, 2 H, H-3, J₁ = 1 Hz, J₂ = 8 Hz), 7.38 (d, 2 H, H-1, J = 2 Hz), 7.02 (m, 4 H, H-2", H-3', H-4'), 6.92 (s (br), 1 H, H-6'), 6.77 (Teil eines AA'BB', 2 H, H-3"), 3.90 (m, 2 H; OCH₂), 2.17 (s, 3 H, Me), 1.80 (m, 1 H), 1.65 (m, 3 H), 1.38 (s (br), 3 H, Me), 1.30 (m, 3 H); 1.16 (m, 3 H), 0.93 (d, 3 H, CH₃, J = 6.6 Hz), 0.86 (d, 6 H; 2 x CH₃, J = 6.7 Hz).

### Beispiel M2: Darstellung von 9-(4-(3,7-dimethyloctyloxy)phenyl)-9-(2,5-dimethylphenyl)fluoren-2,7-bisboronsäure-bisethylenglycolester

Unter Stickstoffatmosphäre wurde (86.0 g, 130 mmol) 2,7-Dibrom-9-(4-(3,7-dimethyloctyloxy)phenyl)-9-(2,5-dimethylphenyl)fluoren in 300 ml destilliertem THF gelöst und unter leichtem Erwärmen zu 7.29 g (300 mmol) Magnesium getropft. Anschließend wurde für 3 h refluxiert. Danach wurde mit 100ml dest THF verdünnt und auf Raumtemperatur abgekühlt. In einem 2 L Kolben wurden 34,3g (330 mmol) Borsäuretrimethylester in 500 ml destilliertem THF gelöst und auf -78°C gekühlt. Bei dieser Temperatur wurde die Grignardlsg. langsam zugetropft, so daß die Temperatur -70°C nicht überschritt (2 Stunden). Über Nacht wurde langsam unter Rühren auf Raumtemperatur erwärmt.
Die galeertartige Masse (grünlich) wurde mit 500 ml Eiswasser und 32,5ml Schwefelsäure conc. versetzt, 60 min gerührt und die organische Phase wurde abgetrennt. Die Wasserphase noch 1 x mit 100 ml Ethylacetat extrahiert.
Die organische Phasen wurden vereinigt mit gesättigter NaCl gewaschen, über MgSO₄ getrocknet, und einrotiert. Es wurden 88,1g Rohprodukt erhalten.
Es wurde nun 2x in 400 ml n-Hexan aufgeschlämmt, 60 min bei RT gerührt, abgesaugt und im Vakuumtrockenschrank bei RT getrocknet (67,5 g Ausbeute).
Die Boronsäure wurde in 450 ml Dichlormethan gelöst, 13 g Ethylenglycol und 0.8 ml Schwefelsäure wurden zugesetzt. Es wurde am Wasserabscheider 5 Stunden zum Rückfluß erhitzt, abgekühlt, mit 100 ml Wasser gewaschen (diese Phasen wurde mit 150 ml Dichlormethan extrahiert) und mit MgSO₄ getrocknet. Es wurde einrotiert und der Rückstand wurde zweimal aus einer Mischung aus 600 ml n-Hexan und 70 ml Essigester umkristallisiert. Man erhielt 24.5 g (32%) 9-(4-(3,7-dimethyloctyloxy)phenyl)-9-(2,5-dimethylphenyl)fluoren-2,7-bisboronsäurebisethylenglycolester als farblose Kristalle mit einer Reinheit (NMR) größer 99%.
¹H NMR (CDCl₃): [ppm] δ = 7.95-7.75 (m, 6H, Fluoren); 7.17 (d, 2H, J = 8 Hz, H-2'); 6.95 (br. s, 1H, H-2"); 6.90 (d, 1H, J = 8 Hz, H-4"); 6.84 (d, 1H, J= 8 Hz, H-5"); 4.34, (s, 8H, Boronsäureester); 3.95-3.85 (m, 2H, OCH₂); 2.20 (s, 3H, CH₃); 1.80-1.45 (m, 4H); 1.35 (3H, CH₃); 1.32-1.10 (m, 6H, Alkyl); 0.90 und 0.85 (2d, 9H, 3 x CH₃).

### 2. Herstellung weiterer Comonomere:

### Beispiel CM1: Darstellung von 2,7-Dibrom-9,9-bis-(2-ethylhexyl)fluoren

Die Darstellung erfolgte in Analogie zu Beispiel 1 in WO 97/05184. Das Produkt (84% Ausbeute) konnte durch doppelte Destillation an einem Kurzwegverdampfer [10⁻³ mbar; 1. Destillation (zum Abtrennen überschüssigen Ethylhexylbromids und restlichem DMSO) 100°C; 2. Destillation: 155°C] als hochviskoses hellgelbes Öl gewonnen werden.
¹H NMR (CDCl₃): [ppm] δ= 7.54 - 7.43 (m, 6H, H-Aryl); 1.93 (d mit Fs., 4 H, *J* = 4.0 Hz); 1.0-0.65 (m, 22H, H-Alkyl); 0.58-0.45 (m, 8H, H-Alkyl).

### Beispiel CM2: Darstellung von 9,9-Bis-(2-ethylhexyl)fluoren-2,7-bisboronsäurebisglykolester

Magnesium (6.32 g, 0.26 mol) wurde in 10 ml THF vorlegt, mit etwas lod versetzt und ein paar Tropfen 2,7-Dibrom-9,9-bis-(2-ethylhexyl)fluoren zugegeben. Das Anspringen der Reaktion war durch starke Exothermie erkennbar. Anschließend wurde parallel die restliche Menge des Bisbromids (insgesamt 68.56 g, 0.125 mol) und 300 ml THF zugetropft. Nach Beendigung der Zugabe wurde für ca. 5 h refluxiert. Es waren nur noch geringe Mengen Mg-Späne zu erkennen.
Parallel dazu wurde Borsäuretrimethylester (28.6 g, 0.27 mol) in THF (200 ml) vorgelegt und auf -70°C gekühlt. Bei dieser Temperatur wurde die Grignardlösung. langsam zugetropft. Anschließend wurde langsam über Nacht unter Rühren auf Raumtemperatur erwärmt.
Die Reaktionslösung wurde auf 300 ml Eiswasser und 10 ml Schwefelsäure conc. gegeben und die organische Phase abgetrennt. Die organische Phase wurde noch einmal mit Wasser gewaschen (neutral). Nach Trocknung über Na₂SO₄ wurde einrotiert. Das Rohprodukt wurde mit Hexan (500 ml) ausgerührt. Dadurch wurde die rohe Bisboronsäure (diese enthält variable Mengen verschiedene Anhydride) erhalten.
Diese wurde direkt durch Refluxieren (12 h) in Toluol mit Ethylenglykol und Schwefelsäure am Wasserabscheider verestert.
Ausbeute über beide Stufen: 70-85%. Reinheit (NMR) >98.5%
¹H NMR (CDCl₃): (NMR-Signale stark verbreitert bzw. verdoppelt wg. Diastereomerie) δ = 7.86 (m, 2 H, H-1); 7.79 (m, 2 H, H-3); 7.73 (d, 2 H, H-4, J = 8 Hz); 4.38 (s (br), 8 H, O-CH₂); 2.02 (m, 4 H, C-CH₂-); 0.75 (m (br), 22 H, H-Alkyl); 0.47 (m (br), 8 H, H-Alkyl).

### Beispiel CM3: Darstellung von 4,4'-Dibromtriphenylamin

Die Darstellung erfolgte analog zu K. Haga *et al*, Bull. Chem. Soc. Jpn., 1986, 59, 803-7: 3.10 g (10.4 mmol) Bis(4-bromphenyl)amin (J. Berthelot *et al*, Can. J. Chem., 1989, 67, 2061), 1.28 g Cyclohexan-1,4-dion (11.4 mmol) und 2.17g (11.4 mmol) p-Toluolsulfonsäure Hydrat wurden in 50 ml Toluol am Wasserabscheider erhitzt. Nach 12 h Reaktionszeit wurde das Lösungsmittel entfernt und durch Säulenchromatographie (Hexan/Essigsäureethylester 4:1) gereinigt. Es wurden 3.82 g (9.46 mmol, 91%) 4,4'-Dibromotriphenylamin als viskoses Öl erhalten.
¹H NMR (CDCl₃): [ppm] δ = 7.01-6.95 (m, 5H), 6.88, 6.74 (AA'BB', 4 + 4 H).

### B) Synthese der Polymere:

### Beispiel P1: Copolymerisation von 9,9-Bis(2-ethylhexyl)fluoren-2,7-bisboronsäurebisglycolester und 2,7-Dibrom-9-(2,5-dimethylphenyl)-9-(4-(3,7-dimethyloctyloxy)phenyl)fluoren durch Suzuki-Reaktion (Polymer P1).

13.21 g (20 mmol) 2,7-Dibrom-9-(2,5-dimethylphenyl)-9-(4-(3,7-dimethyloctyloxy)phenyl)fluoren und 11.61 g K₂CO₃ (84 mmol) wurden in 25 ml Toluol und 25 ml Wasser gelöst und mit N₂ begast. Anschließend wurden 7.743 g (14.6 mmol) 9,9-Bis(2-ethylhexyl)fluoren-2,7-bisboronsäure-bisglykolester sowie 200 mg Pd(PPh₃)₄ (0.17 mmol) unter Schutzgas zugegeben. Die gelb-bräunliche, trübe Suspension wurde unter N₂-Überlagerung bei 87°C Innentemperatur kräftig gerührt. An den folgenden drei Tagen wurde jeweils 1.11 g (2.1 mmol) des Diboronsäureesters zugegeben. Nach 3 Tagen wurde zu der sehr viskosen Mischung weitere 25 ml Toluol zugegeben. Nach insgesamt 4 Tagen wurde aufgearbeitet.

Die Reaktionslösung wurde mit 150 ml Toluol verdünnt, die Lösung wurde mit 200 ml 2% wäßriger NaCN 3h ausgerührt. Dabei hellte sich die Mischung nahezu vollständig auf. Der Ansatz wurde unter Schutzgas in einen Scheidetrichter überführt. Die organische Phase wurde mit H₂O gewaschen und durch Zusetzen in 500 ml Ethanol gefällt.

Das Polymer wurde in 635 ml THF 1 h bei 40°C gelöst und mit 640 ml MeOH ausgefällt, gewaschen und unter Vakuum getrocknet (10.13 g). In 405 ml THF/ 400 ml Methanol wurde ein weiteres Mal umgefällt, abgesaugt und bis zur Massenkonstanz getrocknet. Man erhielt 7.55 g (42 %) des Polymeren P1 als leicht gelben Feststoff.

¹H NMR (CDCl₃): [ppm] δ = 8.1-6.3 (m, 19 H, H-Fluoren, H-Phenyl); 4.0 (m, 2 H, OCH₂); 2.3-0.4 (m, 59 H, Alkyl + Alkoxy-H).
GPC: THF+0.25% Oxalsäure; Säulensatz SDV500, SDV 1000, SDV10000 (Fa. PPS), 35°C, UV Detektion 254 nm: M_{w} = 156000 g/mol, Mₙ = 88000 g/mol.
UV-Vis (Film): λₘₐₓ = 372 nm
PL (Film): λₘₐₓ = 413 nm, 433 nm

### Beispiel P2

### Copolymerisation von 9-(4-(3,7-dimethyloctyloxy)phenyl)-9-(2,5-dimethylphenyl)fluoren-2,7-bisboronsäure-bisethylenglycolester, 2,7-Dibrom-9-(2,5-dimethylphenyl)-9-(4-(3,7-dimethyloctyloxy)phenyl)fluoren und 1 Mol-% 4,4'-Dibromtriphenylamin durch Suzuki-Reaktion (Polymer P2).

6.4733 g (9.8 mmol) 2,7-Dibrom-9-(2,5-dimethylphenyl)-9-(4-(3,7-dimethyloctyloxy)phenyl)fluoren, 6.4246 g (10.00 mmol) 9-(4-(3,7-dimethyloctyloxy)phenyl)-9-(2,5-dimethylphenyl)fluoren-2,7-bisboronsäurebisethylenglycolester, 80.6 mg (0.2 mmol) 4,4'-Dibromtriphenylamin, 9.67 g (42 mmol) K₃PO₄ Hydrat, 30 ml Toluol, 15 ml Wasser und 0.25 ml Ethanol wurden 30 min durch Durchleiten von N₂ entgast. Anschließend wurde 175 mg (0.15 mmol) Pd(PPh₃)₄ unter Schutzgas zugegeben. Die Suspension wurde unter N₂-Überlagerung bei 87°C Innentemperatur (leichter Rückfluß) kräftig gerührt. Nach 4 Tagen wurden weitere 0.30 g 9-(4-(3,7-dimethyloctyloxy)phenyl)-9-(2,5-dimethylphenyl)fluoren-2,7-bisboronsäure-bisethylenglycolester zugesetzt. Nach weiteren 6 Stunden Erhitzen wurden 0.3 ml Brombenzol zugesetzt und noch 3 h zum Rückfluß erhitzt.
Die Reaktionslösung wurde mit 200 ml Toluol verdünnt, die Lösung wurde mit 200 ml 2% wäßrige NaCN 3h ausgerührt. Dabei hellte sich die Mischung nahezu vollständig auf. Die organische Phase wurde mit H₂O gewaschen und durch Zusetzen in 800 ml Ethanol gefällt.

Das Polymer wurde in 200 ml THF 1h bei 40°C gelöst, mit 250 ml MeOH ausgefällt, gewaschen und im Vakuum getrocknet. In 200 ml THF/ 250 ml Methanol wurde ein weiteres Mal umgefällt, abgesaugt und bis zur Massenkonstanz getrocknet. Man erhielt 9.3 g (18.5 mmol, 93 %) des Polymeren P2 als leicht gelben Feststoff.

¹H NMR (CDCl₃): [ppm] δ = 7.8 (m, 2 H, Fluoren); 7.55 (br. s; 4H, Fluoren) 7.15 (br. s, 2H Phenyl); 7.0-6.9 (m, 3H, 2,5-dimethylphenyl); 6.7 (br. s, 2H, Phenyl), 3.95 (br. s, 2H, OCH₂), 2.1 (s, 3H, CH₃); 1.7 (m, 1H, Alkyl); 1,6 (s, 3H, CH₃); 1.5-0.8 (m, 18H, Alkyl).
GPC: THF+0.25% Oxalsäure; Säulensatz SDV500, SDV 1000, SDV10000 (Fa. PPS), 35°C, UV Detektion 254 nm: M_{w} = 43000 g/mol, Mₙ = 23000 g/mol. Elektoluminescenz: λₘₐₓ = 448 nm; Ergebnis bei Max. Eff.: 0.44 cd/A bei 6.7 V/46.9 mA/cm² / 202 cd/m². 100 cd/m² wurden bei einer Spannung von 6.3 V und einer Stromdichte von 24.4 mA/cm² erreicht.

### Beispiel P3

### Polymerisation von 9-(4-(3,7-dimethyloctyloxy)phenyl)-9-(2,5-dimethylphenyl)fluoren-2,7-bisboronsäure-bisethylenglycolester, 2,7-Dibrom-9-(2,5-dimethylphenyl)-9-(4-(3,7-dimethyloctyloxy)phenyl)fluoren durch Suzuki-Reaktion (Polymer P3).

Analog Beispiel P2 wurden 6.6054 g (10.00 mol) 2,7-Dibrom-9-(2,5-dimethylphenyl)-9-(4-(3,7-dimethyloctyloxy)phenyl)fluoren, 6.4246 g (10.00 mmol) 9-(4-(3,7-dimethyloctyloxy)phenyl)-9-(2,5-dimethylphenyl)fluoren-2,7-bisboronsäurebisethylenglycolester, 9.67 g (42 mmol) K₃PO₄ Hydrat in 30 ml Toluol, 15 ml Wasser und 0.25 ml Ethanol unter Zuhilfenahme von 175 mg (0.15 mmol) Pd(PPh₃)₄ polymerisiert Nach Endcapping, analoger Aufarbeitung und Reinigung erhielt man 9.1 g (18.2 mmol, 91 %) des Polymeren P3 als leicht gelben Feststoff.

¹H NMR (CDCl₃): [ppm] δ = 7.8 (m, 2 H, Fluoren); 7.55 (br. s; 4H, Fluoren) 7.15 (br. s, 2H Phenyl); 7.0-6.9 (m, 3H, 2,5-dimethylphenyl); 6.7 (br. s, 2H, Phenyl), 3.95 (br. s, 2H, OCH₂), 2.1 (s, 3H, CH₃); 1.7 (m, 1H, Alkyl); 1,6 (s, 3H, CH₃); 1.5-0.8 (m, 18H, Alkyl).
GPC: THF+0.25% Oxalsäure; Säulensatz SDV500, SDV 1000, SDV10000 (Fa. PPS), 35°C, UV Detektion 254 nm: M_{w} = 47000 g/mol, Mₙ = 27000 g/mol. Elektoluminescenz: λₘₐₓ = 447 nm; Ergebnis bei Max. Eff.: 0.18 cd/A bei 7.2 V/57.3 mA/cm². 100 cd/m² wurden bei einer Spannung von 7.3 V und einer Stromdichte von 62.1 mA/cm² erreicht.

### Beispiel P4

### Polymerisation von 9-(4-(3,7-dimethyloctyloxy)phenyl)-9-(2,5-dimethylphenyl)fluoren-2,7-bisboronsäure-bisethylenglycolester, 2,7-Dibrom-9-(2,5-dimethylphenyl)-9-(4-(3,7-dimethyloctyloxy)phenyl)fluoren und 3% 4,4'-Dibromtriphenylamin durch Suzuki-Reaktion (Polymer P4).

Analog Beispiel P2 wurden 9.328 g (14.10 mmol) 2,7-Dibrom-9-(2,5-dimethylphenyl)-9-(4-(3,7-dimethyloctyloxy)phenyl)fluoren, 7.955 g (15.00 mmol) 9,9-Bis(2-ethylhexyl)fluoren-2,7-bisboronsäure-bisethylenglycolester, 362.8 mg (0.9 mmol) 4,4'-Dibromtriphenylamin, 8.71 g (62 mmol) K₂CO₃ in 30 ml Toluol, 15 ml Wasser und 0.3 ml Ethanol unter Zuhilfename von 260 mg (0.225 mmol) Pd(PPh₃)₄ polymerisiert. Nach Endcapping, analoger Aufarbeitung und Reinigung erhielt man 11.1 g (24.9 mmol, 83 %) des Polymeren P4 als leicht gelben Feststoff.

¹H NMR (CDCl₃): [ppm] δ = 7.85 (m, 1 H Fluoren); 7.75-7.45 (br. m; 4H, Fluoren); 7.28 (m, 1H, Fluoren); 7.1 (br. s, 1 H Phenyl); 7.0-6.9 (m, 1.5H, 2,5-dimethylphenyl); 6.75 (br. s, 2H, Phenyl), 3.95 (br. s, 1H, OCH₂), 2.23 (s, 2H, CH₂); 2.1 -0.5 (m, 27.5 H, Alkyl).

GPC: THF+0.25% Oxalsäure; Säulensatz SDV500, SDV 1000, SDV10000 (Fa. PPS), 35°C, UV Detektion 254 nm: M_{w} = 47000 g/mol, Mₙ = 27000 g/mol. Elektoluminescenz: λₘₐₓ = 446 nm; PL: λₘₐₓ = 425, 452 nm; Ergebnis bei Max. Eff.: 0.36 cd/A bei 7.5 V/74.3 mA/cm²/271 Cd/m². 100 cd/m² wurden bei einer Spannung von 6.6 V und einer Stromdichte von 30.6 mA/cm² erreicht.

### Beispiel P5

### Polymerisation von 9-(4-(3,7-dimethyloctyloxy)phenyl)-9-(2,5-dimethylphenyl)fluoren-2,7-bisboronsäure-bisethylenglycolester, 2,7-Dibrom-9-(2,5-dimethylphenyl)-9-(4-(3,7-dimethyloctyloxy)phenyl)fluoren und 1% 4,4'-Dibromtriphenylamin durch Suzuki-Reaktion (Polymer P5).

Analog Beispiel P2 wurden 12.966 g (19.6 mmol) 2,7-Dibrom-9-(2,5-dimethylphenyl)-9-(4-(3,7-dimethyloctyloxy)phenyl)fluoren, 10.607 g (20.00 mmol) 9,9-Bis(2-ethylhexyl)fluoren-2,7-bisboronsäure-bisethylenglycolester, 161 mg (0.4 mmol) 4,4'-Dibromtriphenylamin, 11.61 g (84 mmol) K₂CO₃ in 40 ml Toluol, 20 ml Wasser und 0.5 ml Ethanol unter Zuhilfename von 350 mg (0.3 mmol) Pd(PPh₃)₄ polymerisiert Nach Endcapping, analoger Aufarbeitung und Reinigung erhielt man 12.2 g (27.4 mmol, 68 %) des Polymeren P5 als leicht gelben Feststoff.

¹H NMR (CDCl₃): [ppm] δ = 7.85 (m, 1 H Fluoren); 7.75-7.45 (br. m; 4H, Fluoren); 7.28 (m, 1 H, Fluoren); 7.1 (br. s, 1 H Phenyl); 7.0-6.9 (m, 1.5H, 2,5-dimethylphenyl); 6.75 (br. s, 2H, Phenyl), 3.95 (br. s, 1 H, OCH₂), 2.23 (s, 2H, CH₂); 2.1 -0.5 (m, 27.5 H, Alkyl).

GPC: THF+0.25% Oxalsäure; Säulensatz SDV500, SDV 1000, SDV10000 (Fa. PPS), 35°C, UV Detektion 254 nm: M_{w} = 53000 g/mol, Mₙ = 31000 g/mol. Elektoluminescenz: λₘₐₓ = 446 nm; PL: λₘₐₓ = 425, 452 nm; Ergebnis bei Max. Eff.: 0.14 cd/A bei 5.7 V/77.0 mA/cm²/110 Cd/m². 100 cd/m² wurden bei einer Spannung von 5.7 V und einer Stromdichte von 83.0 mA/cm² erreicht.

### Vergleichsbeispiele:

### Beispiel V1:

### Suzuki-Polymerisation von 2,7-Dibrom-9,9-bis(2-ethylhexyl)fluoren und 9,9-Bis(2-ethylhexyl)fluoren-2,7-bisboronsäurebisglykolester (Polymer V1), Herstellung von Poly-2,7-[9,9-bis(2-ethylexyl)fluoren]

8.227g (15.00 mmol) 2,7-Dibrom-9,9-bis(2-ethylhexyl)fluoren, 7.956g (15.00 mmol) 9,9-Bis(2-ethylhexyl)fluoren-2,7-bisboronsäurediethylenglykolester, 8.71 g (63 mmol) K₂CO₃, 25 ml Toluol und 15 ml Wasser wurden 30 min durch Durchleiten von N₂ entgast. Anschließend wurden 230 mg (0.2 mmol) Pd(PPh₃)₄ unter Schutzgas zugegeben. Die Suspension wurde unter N₂-Überlagerung bei 87°C Innentemperatur (leichter Rückfluß) kräftig gerührt. Nach 2 Tagen wurden weitere 20 ml Toluol zugegeben, nach weiteren 2 Tagen wurden weitere 0.20 g 9,9-Bis(2-ethylhexyl)fluoren-2,7-bisboronsäurediethylenglykolester zusetzen. Nach weiteren 6 Stunden wurden 0.5 ml 4-Bromfluorbenzol zugesetzt und noch 3 h zum Rückfluß erhitzt.
Die Aufarbeitung erfolgte wie unter Beispiel P1 angegeben. Man erhielt 3.85 g (9.9 mmol, 33%) des Polymers V1 als leicht beigen Feststoff.

¹H NMR (CDCl₃): [ppm] δ = 7.9-7.3 (m, 6 H, H-Arom); 2.15 (br. s, 4H, C(9)-CH₂); 1.1-0.4 (m, 30 H, H-Alkyl).
GPC: THF+0.25% Oxalsäure; Säulensatz SDV500, SDV 1000, SDV10000 (Fa. PPS), 35°C, UV Detektion 254 nm: M_{w} = 70000 g/mol, Mₙ = 34000 g/mol.
UV-Vis (Film): λₘₐₓ = 376 nm
PL (Film): λₘₐₓ = 420 nm, 444 nm

### C) Messungen

Während Polymer V1 in einer typischen EL-Vorrichtung grün-gelbe Emission ergab (Maximum bei ca. 540 nm) zeigte das erfindungsgemäße Polymer P1 kräftig blaue Lumineszenz (Wellenlänge bei ca. 460 nm). Diese Farbe blieb auch während eines längeren Beobachtungszeitraums konstant.

## Patentansprüche

1. Konjugierte Polymere, die Struktureinheiten der Formel (I), 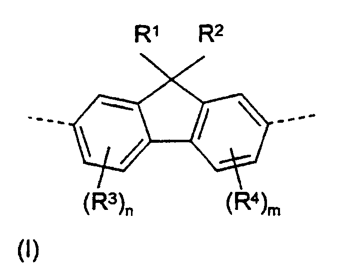 worin
R¹, R² zwei verschiedene Substituenten aus der Gruppe C₂-C₄₀-Hetereoaryl, C₅-C₄₀-Aryl darstellen; wobei die vorstehend genannten Aryle und/oder Heteroaryle mit einem oder mehreren Substituenten R³ substituiert sein können,
R³, R⁴ gleich oder verschieden C₁-C₂₂-Alkyl, C₂-C₂₀-Hetereoaryl, C₆-C₂₀-Aryl, F, Cl, CN, SO₃R⁵, NR⁵R⁶; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein können, wobei die vorstehend genannten Aryle mit einem oder mehreren weiteren nichtaromatischen Substituenten R³ substituiert sein können,
R⁵, R⁶ gleich oder verschieden H, C₁-C₂₂-Alkyl, C₂-C₂₀-Hetereoaryl, C₅-C₂₀-Aryl; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein, wobei die vorstehend genannten Aryle mit einem oder mehreren nichtaromatischen Substituenten R³ substituiert sein können, und
m, n jeweils eine ganze Zahl 0, 1, 2 oder 3 ist, enthalten.

2. Polymer gemäß Anspruch 1, **dadurch gekennzeichnet, daß** es mindestens 10 Mol-% an Struktureinheiten der Formel (I) statistisch, alternierend, periodisch, oder in Blöcken eingebaut enthält.

3. Polymer gemäß Anspruch 1 oder 2, **dadurch gekennzeichnet, daß** es 10 bis 10000 Wiederholeinheiten der Struktureinheit der Formel (I) aufweist.

4. Polymer gemäß einem der Ansprüche 1 bis 3, **dadurch gekennzeichnet, daß** m und n gleich null sind.

5. Verwendung des Polymeren gemäß einem der Ansprüche 1 bis 4 als organischer Halbleiter und/oder als Elektrolumineszenzmaterial.

6. Elektrolumineszenzvorrichtung enthaltend ein Polymer gemäß einem der Ansprüche 1 bis 4.

7. Verbindungen der Formel (A) 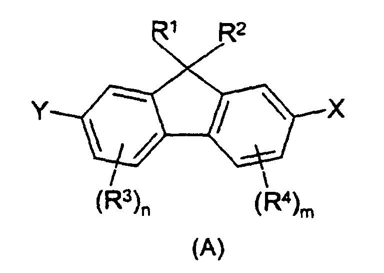 worin
R¹, R² zwei verschiedene Substituenten aus der Gruppe C₂-C₄₀-Hetereoaryl, C₅-C₄₀-Aryl darstellen; wobei die vorstehend genannten Aryle und/oder Heteroaryle mit einem oder mehreren Substituenten R³ substituiert sein können,
R³, R⁴ gleich oder verschieden C₁-C₂₂-Alkyl, C₂-C₂₀-Hetereoaryl, C₅-C₂₀-Aryl, F, Cl, CN, SO₃R⁵, NR⁵R⁶; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein können, wobei die vorstehend genannten Aryle mit einem oder mehreren nichtaromatischen Substituenten R³ substituiert sein können,
R⁵, R⁶ gleich oder verschieden H, C₁-C₂₂-Alkyl, C₂-C₂₀-Hetereoaryl, C₅-C₂₀-Aryl; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein, wobei die vorstehend genannten Aryle mit einem oder mehreren nichtaromatischen Substituenten R³ substituiert sein können, und
m, n jeweils eine ganze Zahl 0, 1, 2 oder 3 ist,
X, Y gleich oder verschieden Halogen, B(OR⁷)₂ oder SnR⁷R⁸R⁹
R⁷, R⁸, R⁹ gleich oder verschieden H, C₁-C₆-Alkyl, wobei zwei Reste auch einen gemeinsamen Ring bilden können und diese Reste auch verzweigt oder unverzweigt sein können.

## Claims

1. Conjugated polymers, which contain structural units of the Formula (1) 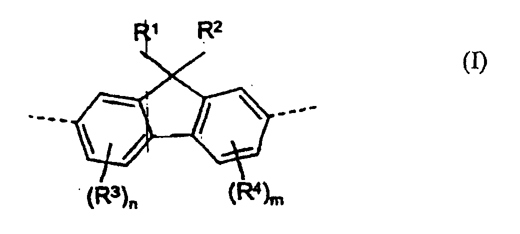 where
R¹, R² represent two different substituents from the group C₂-C₄₀-heteroaryl, C₅-C₄₀-aryl; whereby the above-mentioned aryls and / or heteroaryls may be substituted with one or more substituents R³
R³, R⁴ represent the same or different members of C₁-C₂₂-alkyl, C₂-C₂₀-heteroaryl, C₅-C₂₀-aryl, F, Cl, CN, SO₃R⁵, NR⁵R⁶ when the alkyl residues may be branched or unbranched or may, also, be cycloalkyls; and single, non-adjacent CH₂-groups of the alkyl residue can be replaced by O, S, C=O, COO, N-R⁵ or even simple aryls, whereby the above-named aryls may be substituted with one or more other non-aromatic substituents R³
R⁵, R⁶ represent the same or different members of H, C₁-C₂₂-alkyl, C₂-C₂₀-heteroaryl, C₅-C₂₀-aryl: the alkyl residues may be branched or unbranched or may, also, be cycloalkyls; and single, non-adjacent CH₂-groups of the alkyl residue can be replaced by O, S, C=O, COO, N-R⁵ or even simple aryls, whereby the above-named aryls may be substituted with one or more other non-aromatic substituents R^{3,} and
m, n are each whole numbers, i.e 0, 1, 2 or 3.

2. Polymer in accordance with claim 1, **characterised in that** it contains at least 10 Mol % of structural units in accordance with Formula (1) distributed randomly, alternately, periodically or in blocks.

3. Polymer in accordance with claims 1 or 2, **characterised in that** it exhibits 10 to 10,000 repeat units of the structural unit represented by Formula (I).

4. Polymer in accordance with one of the claims 1 to 3, **characterised in that** both n and m are equal to zero.

5. Use of the polymer in accordance with one of claims 1 to 4 as an organic semi-conductor and / or as an electro-luminescent material.

6. Electro-luminescent device containing a polymer in accordance with one of claims 1 to 4.

7. Compounds of the Formula (A) 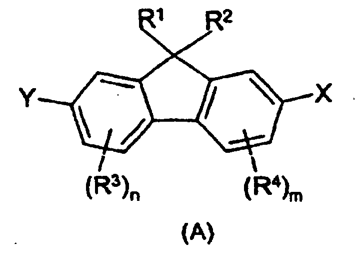 where
R¹, R² represent two different substituents from the group C₂-C₄₀-heteroaryl, C₅-C₄₀-aryl; whereby the above-mentioned aryls and / or heteroaryls may be substituted with one or more substituents R³
R³, R⁴ represent the same or different members of C₁-C₂₂-alkyl, C₂-C₂₀-heteroaryl, C₅-C₂₀-aryl, F, Cl, CN, SO₃R⁵, NR⁵R⁶ when the alkyl residues may be branched or unbranched or may, also, be cycloalkyls; and single, non-adjacent CH₂-groups of the alkyl residue can be replaced by O, S, C=O, COO, N-R⁵ or even simple aryls, whereby the above-named aryls may be substituted with one or more other non-aromatic substituents R³
R⁵, R⁶ represent the same or different members drawn from H, C₁-C₂₂-alkyl, C₂-C₂₀-heteroaryl, C₅-C₂₀-aryl: the alkyl residues may be branched or unbranched or may, also, be cycloalkyls; and single, non-adjacent CH₂-groups of the alkyl residue can be replaced by O, S, C=O, COO, N-R⁵ or even simple aryls, whereby the above-named aryls may be substituted with one or more other non-aromatic substituents R^{3,} and
m, n are each whole numbers, i.e 0, 1, 2 or 3.
X, Y are the same or different halogens, B(OR⁷)₂ or SnR⁷R⁸R⁹
R⁷, R⁸, R⁹ are similar or different members of H, C₁-C₆-alkyl, where two residues can also form a common ring and these residues may be either branched or unbranched.

## Revendications

1. Polymères conjugués, qui répondent aux unités de structure de formule (I) 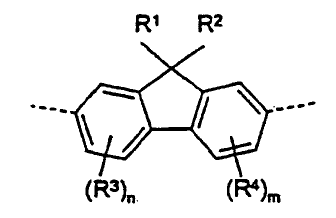 où
R¹, R² représentent deux substituants différents pris parmi les groupes hétéroaryle en C₂-C₄₀, aryle en C₅-C₄₀ ; les groupes aryle et/ou hétéroaryle précités peuvent être substitués par un ou plusieurs substituants R³,
R³, R⁴ identiques ou différents représentent un groupe alkyle en C₁-C₂₂, hétéroaryle en C₂-C₂₀, aryle en C₅-C₂₀, F, Cl, CN, SO₃R⁵, NR⁵R⁶ ; dans ce contexte, les restes alkyles peuvent être ramifiés ou non ramifiés ou représenter aussi un groupe cycloalkyle ; et les différents groupes CH₂ non adjacents du reste alkyle peuvent être remplacés par O, S, C=O, COO, N-R⁵ ou aussi par des groupes aryles simples, les groupes aryles précités peuvent être substitués par un ou plusieurs substituants non aromatiques R³,
R⁵, R⁶ identiques ou différents représentent un atome d'hydrogène, un groupe alkyle en C₁-C₂₂, hétéroaryle en C₂-C₂₀, aryle en C₅-C₂₀ ; les restes alkyle peuvent être ramifiés ou non ramifiés ou aussi représenter un groupe cycloalkyle ; et les différents groupes CH₂ non adjacents du reste alkyle peuvent être remplacés par O, S, C=O, COO, N-R⁵ ou aussi par des groupes aryles simples, les groupes aryles précités peuvent être substitués par un ou plusieurs substituants non aromatiques R³, et
m, n sont chacun un entier 0, 1, 2 ou 3.

2. Polymère selon la revendication 1, **caractérisé en ce qu'**au moins 10 % molaires d'unités de structure de formule (I) sont incorporés statistiquement, en alternance, périodiquement ou en blocs.

3. Polymère selon la revendication 1 ou 2, **caractérisé en ce qu'**il présente 10 à 10000 unités récurrentes de l'unité de structure de formule (I).

4. Polymère selon l'une des revendications 1 à 3, **caractérisé en ce que** m et n sont égaux à zéro.

5. Utilisation du polymère selon l'une des revendications 1 à 4 en tant que semi-conducteur organique et/ou comme matière électroluminescente.

6. Dispositif électroluminescent renfermant un polymère selon l'une des revendications 1 à 4.

7. Composés de formule (A) 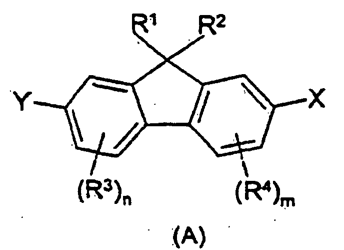 où
R¹, R² représentent deux substituants différents pris parmi les groupes comprenant hétéroaryle en C₂-C₄₀, aryle en C₅-C₄₀ ; les groupes aryle et/ou hétéroaryle précités peuvent être substitués par un ou plusieurs substituants R³,
R³, R⁴ identiques ou différents représentent un groupe alkyle en C₁-C₂₂, hétéroaryle en C₂-C₂₀, aryle en C₅-C₂₀, F, Cl, CN, SO₃R⁵, NR⁵R⁶ ; dans ce contexte, les restes alkyles peuvent être ramifiés ou non ramifiés ou représenter aussi un groupe cycloalkyle ; et les différents groupes CH₂ non adjacents du reste alkyle peuvent être remplacés par O, S, C=O, COO, N-R⁵ ou aussi par des groupes aryles simples, les groupes aryles précités peuvent être substitués par un ou plusieurs substituants non aromatiques R³,
R⁵, R⁶ identiques ou différents représentent un atome d'hydrogène, un groupe alkyle en C₁-C₂₂, hétéroaryle en C₂-C₂₀, aryle en C₅-C₂₀ ; les restes alkyle peuvent être ramifiés ou non ramifiés ou aussi représenter un groupe cycloalkyle ; et les différents groupes CH₂ non adjacents du reste alkyle peuvent être remplacés par O, S, C=O, COO, N-R⁵ ou aussi par des groupes aryles simples, les groupes aryles précités peuvent être substitués par un ou plusieurs substituants non aromatiques R³, et
m, n sont chacun un entier 0, 1, 2 ou 3.
X, Y identiques ou différents représentent un atome d'halogène, un groupe B(OR⁷)₂ ou SnR⁷R⁸R⁹,
R⁷, R⁸, R⁹ identiques ou différents représentent un atome d'hydrogène, un groupe alkyle en C₁-C₆, deux restes peuvent former également un cycle commun et ces restes peuvent aussi être ramifiés ou non ramifiés.
